Europäisches Patentamt

European Patent Office

Office européen des brevets

(11) Publication number: **0 006 545**
A2

(19)

## EUROPEAN PATENT APPLICATION

(12)

(21) Application number: 79101948.2

(51) Int. Cl.³: **A 61 M 31/00**

(22) Date of filing: 15.06.79

(30) Priority: 27.06.78 IT 2218478

(43) Date of publication of application: 09.01.80
Bulletin 80/1

(84) Designated Contracting States: **AT BE CH DE FR LU NL
SE**

(71) Applicant: **Boschetti, Enrica, Via Melchiorre Gioia,
I-171-20125 Milano (IT)**

(72) Inventor: **Boschetti, Enrica, Via Melchiorre Gioia,
I-171-20125 Milano (IT)**

(74) Representative: **Ferraiolo, Ruggero, Viale Tunisia, 29,
I-20124 Milano (IT)**

(54) **A set for containing and applying into a human anatomic cavity a metered quantity of doughy medical or contraceptive substance.**

(57) A set for containing and applying into a human anatomic cavity a metered quantity of doughy medical or contraceptive substance consisting of an expendable cannula (1) and of a piston carrying rod (7). The cylindrical hollow (2) of the cannula is filled in with the medical or contraceptive substance and is closed at both ends (10, 11) by easily removable closing means (3, 5). At the moment of using the set, the closing means are removed by the fingers, the piston (9) is introduced into the proper end (10) of the hollow (2), the cannula (1) is introduced into the anatomic cavity and the rod (7) is pushed along the hollow (2) untill all the substance is ejected (see figure 3).

- 1 -

## A set for containing and applying into a human anatomic cavity a metered quantity of doughy medical or contraceptive substance.

The invention relates to a set for containing and applying into a human anatomic cavity a metered quantity of doughy medical or contraceptive substance; a hollow cannula acts as a container of the substance and a separate rod provided with a piston is suitable to be pushed into the hollow of the cannula in order to eject the substance after the introduction of the cannula into the cavity.

There are doughy medical or contraceptive substances which are contained in tubes, as tubes for tooth-paste, ointments, cosmetic creams, and are to be introduced into bottom anatomic parts, as the vaginal or anal cavity. Such substances packed in said tubes are generally applied by the following known means and procedure: the tube is sold together with a separate metering cannula which is provided with a piston; the cannula is fitted for the tube nozzle in such a way that, if the tube is pressed, a required quantity of substance is removed into the cannula; the cannula is disjoined from the tube nozzle and conveniently introduced into the anatomic cavity; then a piston is applied and pushed into the cannula

Dott. Ing. Ruggero Ferraiolo

- 2 -

in order to ejected the substance.

The disadvantages of the above known means are that (1) the cannula is used a number of times and consequently must be made of durable and relatively expensive material, (2) the cannula must be carefully cleaned and put away after every use and (3) these means are cumbersome and not easy for the user since the cannula must be filled in at the moment of applying the substance and also the tube must be taken with oneself.

The invented set consists of an expendable cannula containing a metered quantity of substance and of a piston carrying rod that, at the moment of using this set, is introduced into the cannula and is pushed so as to eject all the contained substance in the proper anatomic cavity wherein the cannula is introduced.

The cannula ends are closed by two convenient means which may be easily removed: the end into which the piston is to be introduced is closed by a plug that extends into the cannula by a short length so that, once the plug is removed, the piston is introduced into the cannula short length which is not filled in with the substance; the opposite end is closed by a small cap.

Of course, the cannula will be of material and sizes suitable to the purpose, namely the material will be chemically suitable to the contained substance, the length and the outer diameter will be suitable to the prescribed anatomic cavity and the capacity will be adequate with the required quantity

Dott. Ing. Ruggero Ferraiolo

- 3 -

of substance. The length of the rod comprising the piston will be substantially equal with the length of the cannula and the piston diameter will be adequate with the cannula hollow.

It will be appreciated that the invented set may be used in a easy and rapid manner; it is handy since the cannula and the piston carrying rod may be thrown away after use; it is cheap because it is made of non durable material and, if desired, the piston bearing rod may be kept for further use.

For a better understanding of the invented set, an embodiment thereof will be illustrated, as an example only, with reference to the accompanying drawings in which:

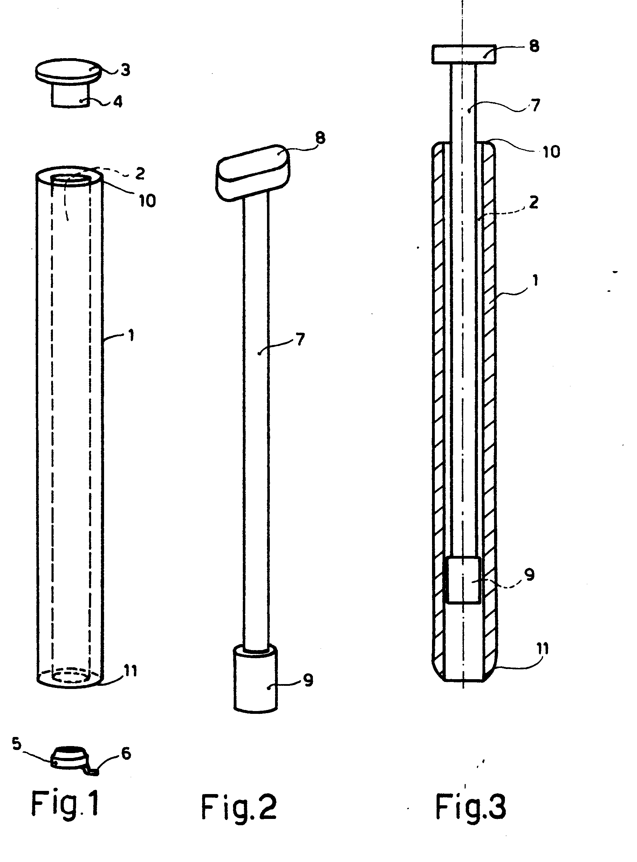

Figure 1 is a view of a cannula and of the separate clo sing means,

Figure 2 is a view of the piston carrying rod,

Figure 3 shows the cannula into which the piston carrying rod is introduced partly.

Such set consists of a cylindrical cannula 1 made of suitable plastic material which comprises a cylindrical hollow 2; of the upper plug 3 and lower cap 5; of the rod 7 which is made of suitable plastic material and is provided at the ends with a piston 9 and a grip 8 integral with the rod 7.

The upper plug 3 is provided with an extension 4 which penetrates the hollow 2 by a short length. The cap 5 is provided

Dott. Ing. Ruggero Ferraiolo

- 4 -

with a small tongue 6 which makes easier the removing of the cap itself from the hollow 2.

In the workshop the cannula shall be filled in with the proper quantity of substance and then shall be closed at the end 10 by the plug 3 and at the end 11 by the cap 5.

The diameter of the piston 9 is such that it fits conveniently the hollow 2 of cannula 1; the length of the rod 7 added to that of the piston 9 is substantially equal with the length of the cannula 1; finally, the edge of the end 11 of cannula 1 is conveniently bevelled in order to facilitate the introducing of the cannula into the anatomic cavity. Thus, it will be appreciated that, to use such set and to apply the substance contained therein, it is necessary to remove the plug 3 and the cap 5, to insert the piston 9 into the empty part as let by the extension 4 into the hollow 2, to penetrate the cannula 1 into the prescribed anatomic cavity and to push the rod 7 towards the end 11 of the cannula; in such a way all the substance is ejected from the cannula.

Dott. Ing. Ruggero Ferraiolo

- 1 -

## CLAIM

A set for containing and applying into a human anatomic cavity a metered quantity of doughy medical or contraceptive substance caracterized in that it consists of an expendable cannula (1), provided with a longitudinal cylindrical hollow (2), which is suitable to contain a metered quantity of the substance to be applied, is suitable to be introduced into a prescribed anatomic cavity and is provided at both ends (10, 11) with easily removable closing means (3, 5) as well as of a corresponding rod (7) provided with a grip (8) at one end and with a piston (9) conveniently fitting the hollow (2) of the cannula (1) at the other end such that, by pushing the rod (7) into the hollow (2), all the substance contained therein is ejected.

Dott. Ing. Ruggero Ferraiolo

0006545

— 1/1 —

Fig.1    Fig.2    Fig.3

Dott. Ing. Ruggero Ferraiolo